# EUROPEAN PATENT APPLICATION

(11) **EP 0 974 362 A1**
(43) Date of publication of application: **26.01.2000**
(21) Application number: 98907157.6
(22) Date of filing: 11.03.1998
(51) Int. Cl.: A61K 45/00, A61K 31/70

(54) **DRUGS CONTAINING ENZYME INHIBITORS AS THE ACTIVE INGREDIENT**

(30) Priority: 13.03.1997 JP 5906597
(71) Applicant: CHUGAI SEIYAKU KABUSHIKI KAISHA, Tokyo, 115-8543 (JP)
(72) Inventor: KAMATANI, Naoyuki, Tokyo Women's Medical College, Tokyo 162-0054 (JP); DAN, Takashi, Chugai Seiyaku Kabushiki Kaisha, Gotenba-shi, Shizuoka 412-8513 (JP); OSADA, Hiroshi, Chugai Seiyaku Kabushiki Kaisha, Gotenba-shi, Shizuoka 412-8513 (JP)
(74) Representative: VOSSIUS & PARTNER
(86) International application number: JP9800997
(87) International publication number: WO9840101

(57) **Abstract**

Drugs capable of selectively inhibiting the proliferation or metabolism of target cells such as cancer cells without affecting normal cells. Such a drug contains as the active ingredient an enzyme inhibitor and satisfies the following requirements: 1) the enzyme inhibitor being capable of inhibiting at least one enzyme essentially required in the first metabolic pathway of a compound essentially required in the survival of cells (i.e., an essential compound); 2) the essential compound being metabolizable in a second pathway which is different from the first pathway and not inhibited by the enzyme inhibitor; and 3) at least one enzyme essentially required in the second metabolic pathway lacking its function in the target cells the proliferation of which is to be inhibited by the drug.

## Description

### TECHNICAL FIELD

The present invention relates to drugs targeted at cells functionally deficient in an enzyme of one pathway (first enzyme) related to metabolism of a compound essential for cell survival as well as methods and kits for testing the potency of said drugs, more specifically to drugs acting to inhibit an enzyme of another pathway (second enzyme) complementing the function of said first enzyme as well as methods and kits for testing the potency of said drugs, comprising determining that said first enzyme is functionally lost.

### BACKGROUND ART

Many of conventional anticancer drugs are targeted at the enhanced growth or metabolic potency in cancer cells and include various types such as antimetabolites, DNA modifiers, inhibitors of cell division, hormone inhibitors, topoisomerase inhibitors. However, none of them acts specifically on cancer cells, but they show similar effects on normal cells under active growth or metabolism such as bone marrow cells, gut epithelial cells, hair follicular matrix cells, thereby frequently causing serious side effects. Thus, no anticancer drug that inhibits only the growth or metabolism of cancer cells with high selectivity without influencing normal cells has been found.

Recent developments of analytic techniques of human genomes have rushed genetic analyses of cancer cells by combinations of Southern blotting, Polymerase Chain Reaction (PCR), DNA sequencing or the like to reveal some loci on genes associated with mutations at high frequency in cancer cells. Mutations include various forms such as point mutation, deletion, duplication, inversion, insertion, translocation, methylation, which may result in functional deficiency in some enzymes. For example, malignant melanoma, glioma, lung cancer, leukemia or other diseases often show deletions at chromosomal locus 9p21, particularly deletions of the gene for the cyclin-dependent kinase 4 inhibitor CDK4I (p16) and the neighboring gene for 5'-methylthioadenosine phosphorylase (hereinafter abbreviated as MTAP) at high frequency (Nature, 368, 753-756, 1994). Human pancreatic cancer strains also show deletions of p16 at high frequency simultaneously with functional deficiency in MTAP (Cancer Res., 56, 1083-1090, 1996). Functional deficiency in MTAP associated with gene deletion has also been reported in mammary cancers and bladder cancers (Proc. Natl. Acad. Sci. USA, 93, 6203-6208, 1996).

MTAP is an enzyme that converts 5'-methylthioadenosine (hereinafter abbreviated as MTA) produced in conjugation with polyamine synthesis into adenine, and it is essential for the salvage pathway for ATP recycling, so that cancer cells lacking this enzyme can utilize only the de novo pathway for purine nucleotide synthesis.

Such deficiency in some enzymes associated with mutations of genes can be used for selective toxicity. In MTAP-deficient cancers, for example, cancer cells can not survive but normal cells having a salvage pathway can survive by specifically inhibiting the de novo pathway of purine nucleotide synthesis. In fact, growth of MTAP-deficient cancers can be selectively suppressed by inhibiting the de novo pathway of purine nucleotide synthesis with a folate analogue methotrexate or an L-glutamine antagonist azaserine (Proc. Natl. Acad. Sci. USA, 78, 1219-1223, 1981), but these drugs disadvantageously have a wide range of actions to cause side effects or drug resistance ("Antimetabolites" in The Anticancer Drugs 2nd ed., New York: Oxford Univ. Press, 69-107, 1994; Pharmac. Ther., 46, 243-271, 1990).

### DISCLOSURE OF THE INVENTION

As a result of careful studies to seek a drug selectively inhibiting the growth or metabolism of target cells such as cancer cells without influencing normal cells, the present inventors found a drug targeted at cells functionally deficient in an enzyme of one pathway (first enzyme) related to metabolism of a compound essential for cell survival as well as a method and a kit for testing the potency of said drug, more specifically, a drug acting to inhibit an enzyme of another pathway (second enzyme) complementing the function of said first enzyme and a method and a kit for testing the potency of said drug, comprising determining that said first enzyme is functionally lost in target cells, and thus attained the present invention.

According to the first aspect of the present invention, a drug containing an enzyme inhibitor as an active ingredient is provided, wherein said enzyme inhibitor satisfies the following requirements:
1) said enzyme inhibitor inhibits at least one enzyme which is essential for one metabolic pathway (first pathway) of a compound essential for cell survival (essential compound);
2) said essential compound is also metabolizable by another pathway (second pathway) which is different from said first pathway and said enzyme inhibitor does not inhibit said second pathway; and
3) at least one enzyme essential for said second pathway is functionally lost in a target cell subjected to growth inhibition by said drug.

According to the second aspect of the present invention, a method for testing the potency of a drug containing an enzyme inhibitor as an active ingredient is provided, wherein said enzyme inhibitor inhibits at least one enzyme which is essential for one metabolic pathway (first pathway) of a compound essential for cell survival (essential compound), and said essential compound is also metabolizable by another pathway (second pathway) which is different from said first pathway, and said enzyme inhibitor does not inhibit said second pathway.

According to the third aspect of the present invention, a kit for testing the potency of a drug containing an enzyme inhibitor as an active ingredient is provided, wherein said enzyme inhibitor inhibits at least one enzyme essential for one metabolic pathway (first pathway) of a compound essential for cell survival (essential compound), and said essential compound is also metabolizable by another pathway (second pathway) which is different from said first pathway, and said enzyme inhibitor does not inhibit said second pathway.

### BRIEF DESCRIPTION OF THE DRAWINGS

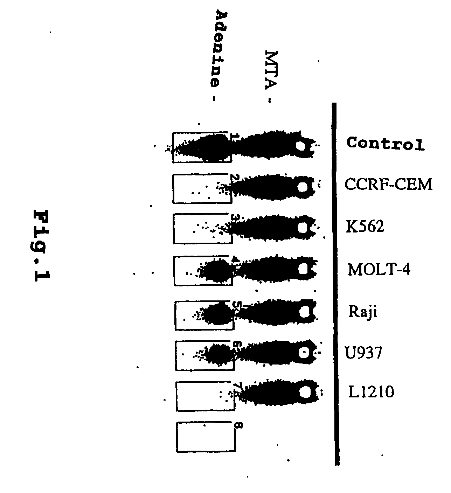

Fig. 1 shows 5'-methylthioadenosine phosphorylase (MTAP) activity of human leukemia and lymphoma cell lines. Cell extracts were reacted with [8-¹⁴C] MTA, a substrate for MTAP, and the reaction solutions were partially developed by thin-layer chromatography to visualize spots corresponding to [¹⁴C] adenine produced by the action of MTAP in the presence of [8-¹⁴C] MTA (shown in frames) with a FUJIX Bio-Imaging Analyzer BAS 2000.

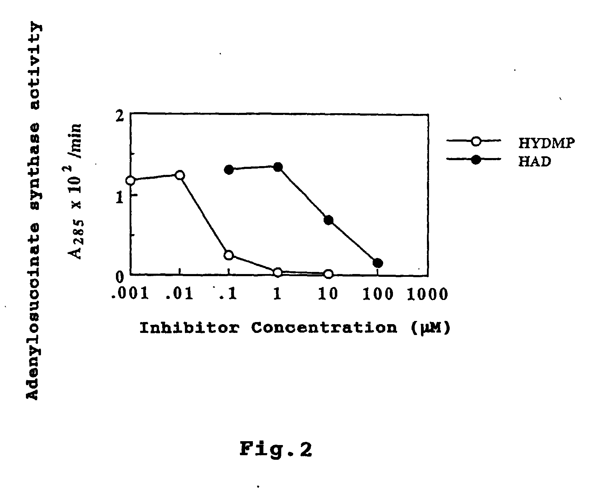

Fig. 2 shows inhibition of adenylosuccinate synthase activity by hydantocidin-5'-monophosphate (abbreviated as HYDMP) and hadacidin (abbreviated as HAD). The abscissa indicates concentrations of the inhibitors and the ordinate indicates change of absorbance at 285 nm with time for a period of 2 to 5 minutes after the reaction is started.

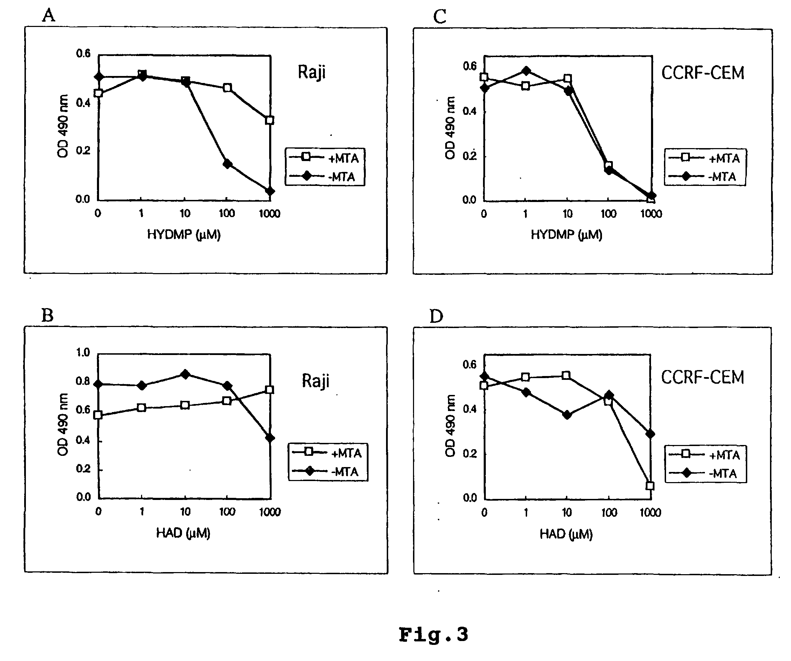

Fig. 3 shows effects of adenylosuccinate synthase inhibitors [hydantocidin-5'-monophosphate (HYDMP) and hadacidin (HAD)] on the growth of Raji cells having MTAP activity (A, B) or CCRF-CEM cells lacking MTAP activity (C, D). The abscissa indicates concentrations of the inhibitors and the ordinate indicates absorbances at 490 nm corresponding to survival cell number.

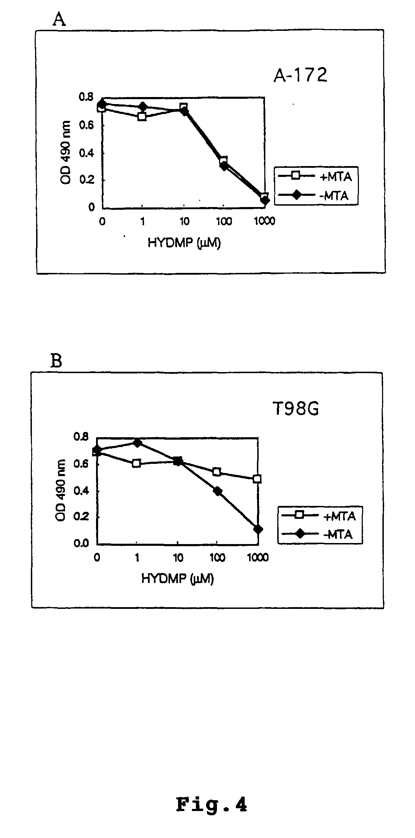

Fig. 4 shows effects of an adenylosuccinate synthase inhibitor hydantocidin-5'-monophosphate (HYDMP) on the growth of A-172 cells lacking MTAP activity (A) and T98G cells having MTAP activity (B). The same experiment was performed as for Raji cells in Fig. 3 except that incubation was performed for 3 days. The abscissa indicates concentrations of the inhibitor and the ordinate indicates absorbances at 490 nm corresponding to survival cell number.

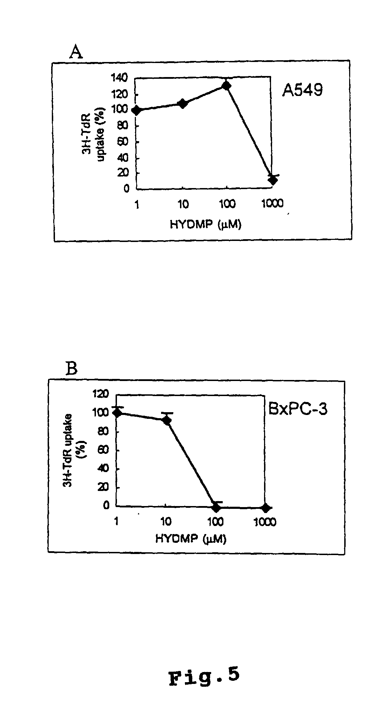

Fig. 5 shows effects of an adenylosuccinate synthase inhibitor hydantocidin-5'-monophosphate (HYDMP) on the growth of A549 cells (A) and BxPC-3 cells (B) lacking MTAP activity. The same experiment was performed as for Raji cells in Fig. 3 except that incubation was performed for 3 days and that [³H] thymidine uptake for 5 hours was assayed. The abscissa indicates concentrations of the inhibitor and the ordinate indicates radioactivities incorporated into cells in percent (% vs. the level in the absence of HYDMP).

### PREFERRED EMBODIMENT FOR CARRYING OUT THE INVENTION

As used herein, the first pathway of a compound essential for cell survival (essential compound) means one of two pathways for the metabolism of the essential compound when the function of at least one enzyme essential for another pathway is lost or greatly decreased in target cells such as cancer cells. Such a pathway includes, for example, a pathway for AMP biosynthesis from IMP through adenylosuccinic acid to AMP (de novo pathway). In this case, the enzyme essential for the first pathway includes an enzyme essential for biosynthesis of adenylosuccinic acid from IMP, adenylosuccinate synthase (hereinafter abbreviated as AdSS) or the like.

As used herein, the metabolism means both biosynthesis and degradation.

The second pathway of the essential compound means a metabolic pathway involving an enzyme of which the function is lost or greatly decreased in target cells such as cancer cells but sufficiently effective in normal cells. Preferably, such a pathway is suitable for readily determining the presence or absence of the activity of the enzyme or readily determining the presence or absence of the enzyme by genetic diagnosis. Such a pathway includes, for example, the salvage pathway for AMP synthesis. The enzyme essential for the second pathway includes MTAP, which converts MTA into adenine, or the like.

The target cells mean cells to be treated by a drug of the present invention, such as cancer cells. The target cells as used herein must be cells lacking the function of the enzyme essential for the second pathway of an essential compound, such as the human leukemia cell line CCRF-CEM, the human glioma cell line A-172, the human non-small cell lung cancer cell line A549, the human pancreatic cell line BxPC-3, etc. The expression that an enzyme is functionally lost in these target cells means that the essential compound can not be metabolized or can be metabolized only at a very low efficiency by mutation of the enzyme gene. Drugs of the present invention are more effective when the enzyme itself is wholly lost particularly due to homozygous deletion of the enzyme gene or the like.

As used herein, the compound essential for cell survival (essential compound) means a nucleotide or a nucleotide metabolite, such as AMP, ADP, ATP, GMP, GDP, GTP, UMP, UDP, UTP, CTP, dTMP.

As used herein, the compound serving as a substrate for at least one enzyme essential for the second pathway means a compound serving as a substrate for the compound metabolized by said enzyme and which can be added to further enhance effects of drugs of the present invention. Namely, said compound can be added to induce metabolism of an essential compound by the action of an enzyme essential for the second pathway so that normal cells can survive or can be salvaged but target cells functionally lacking the second enzyme can not survive because the metabolism of the essential compound is not induced. Such a compound serving as a substrate includes, for example, MTA.

When a plurality of enzymes for the first pathway complementing the function of an enzyme essential for the second pathway exist, an enzyme determining the rate of the alternative function is preferably selected as an enzyme essential for the first pathway. For example, in case of the de novo pathway for purine nucleotide synthesis complementing the function of MTAP, which involves a sequence of reactions mediated by a plurality of enzymes, it is desirable to select AdSS which rate-determines AMP synthesis at the branch point of AMP synthesis as an enzyme essential for the first pathway.

The inhibitor of the enzyme essential for the first pathway is desirably selected by an activity assay system with a high specificity for said enzyme. For example, activity of AdSS can be quantified by determining change of UV absorption spectrum with time as adenylosuccinic acid consisting of a substrate inosinic acid linked to another substrate aspartic acid is produced (Curr. Top. Cell. Regul., 22, 103-141, 1983). A method determining the level of radioactively labeled aspartic acid converted into adenylosuccinic acid (Cancer Res., 40, 4390-4397, 1980) is also available.

The inhibitor of the enzyme essential for the first pathway desirably shows selective toxicity to target cells lacking an enzyme essential for the second pathway. Cells containing the enzyme essential for the second pathway and cells lacking it can be used to determine whether or not the inhibitor of the enzyme essential for the first pathway selectively acts on target cells lacking the enzyme essential for the second pathway. For example, MTAP-deficient cancer cells include the human leukemia cell line CCRF-CEM, the human glioma cell line A-172, the human non-small cell lung cancer cell line A549, the human pancreatic cell line BxPC-3, etc. Generally, they are especially often found in human leukemia cells, human glioma cells, human melanoma cells, human lung cancer cells, human pancreatic cancer cells, etc. Controls with MTAP include the human lymphoma cell line Raji, the human glioma cell line T98G, etc. The selectivity of cytotoxicity can be determined by adding a substrate for MTAP, MTA into the medium.

Such an inhibitor includes, for example, AdSS inhibitors such as hydantocidin or derivatives thereof, hadacidin or the like. Hydantocidin derivatives include, for example, hydantocidin-5'-monophosphate. These compounds are known and can be prepared by the process described in literatures (Tetrahedron, 47, 2133-2144, 1991; Proc. Natl. Acad. Sci. USA, 93. 9431-9436, 1996), for example.

The present invention also includes a method and a kit for testing the potency of a drug of the present invention to patients by determining whether or not the drug can inhibit the growth or metabolism of a target cell. More specifically, a method and a kit for testing the potency of a drug containing an enzyme inhibitor as an active ingredient is included, wherein said enzyme inhibitor inhibits at least one enzyme essential for one metabolic pathway (first pathway) of a compound essential for cell survival (essential compound), and said essential compound is also metabolizable by another pathway (second pathway) which is different from said first pathway, and said enzyme inhibitor does not inhibit said second pathway, said method or kit comprising determining that at least one enzyme essential for the second pathway of said essential compound is functionally lost only in a target cell subjected to growth inhibition by said drug.

In the method of the present invention, means for determining that at least one enzyme essential for the second pathway of said essential compound is functionally lost only in a target cell subjected to growth inhibition by said drug includes a procedure for detecting mutation presumed to be the cause of the functional deficiency in said enzyme such as homozygous deletion of a gene, or a procedure for determining the presence or absence of the activity of said enzyme, specifically the following procedure, for example.

Target cells such as cancer cells are disrupted by freezing and thawing or with a surfactant such as Triton X-100, Nonidet P-40 to prepare crude extracts and optionally to partially purify fractions containing an enzyme essential for the second pathway. An activity assay highly specific for said enzyme is used to detect whether or not the functions of said enzyme are normal. In case of MTAP, for example, conversion of a radioactively labeled substrate MTA into a reaction product adenine can be detected by thin-layer chromatography (see Biochem. Biophys. Res. Commun., 95, 1861-1866, 1980; Proc. Natl. Acad. Sci. USA, 78, 1219-1223, 1981; WO95/18233).

An antibody specifically directed to an enzyme essential for the second pathway may be used to detect the presence or absence of said enzyme. For example, an antibody specifically directed to MTAP can be used to determine the presence or absence of MTAP in human glioma cell extracts (see Cancer Res., 51, 3193-3197, 1991; WO95/18233).

On the other hand, homozygous deletion of the gene for an enzyme essential for the second pathway in target cells can be detected by preparing genomic DNA extracted from the target cells and performing PCR using oligo DNA fragments corresponding to the DNA sequences surrounding the gene encoding said enzyme, wherein any homozygous deletion is indicative of functional deficiency in said enzyme. These primers can be used to construct a kit for testing drug potency. For example, DNA sequences surrounding the gene can also be used in case of MTAP deletion to detect homozygous deletion (see Nature, 368, 753-756, 1994; WO95/18233).

However, determination is desirably based on the presence or absence of activity or using an antibody, because enzymatic deficiency is caused by not only homozygous deletion but also other events which can not necessarily be detected by the PCR method, such as point mutation, frameshift mutation, translocation, variation of expression regulatory regions, variation of expression regulatory proteins, etc.

Preferably, drugs and diagnostic methods of the present invention are particularly used as anticancer drugs or cancer diagnosis, but also can be applied to any diseases related to genetic deficiency.

The whole content of Japanese Patent Application No. 59065/97, on which the priority claim of the present application is based, is incorporated herein as reference.

The following examples further illustrate the present invention, however, without limiting the same thereto.

### Examples

### Example 1: Detection procedure of 5'-methylthioadenosine phosphorylase (MTAP)-deficient cancers

Cells of human leukemia cell lines (CCRF-CEM, K-562, MOLT-4) and human lymphoma cell lines (Raji, U937) suspended in Dulbecco's phosphate buffer were disrupted by repeating freezing and thawing, and centrifuged to give cell extracts. To 10 µl of each cell extract diluted at a protein concentration of 1.2 mg/ml with Dulbecco's phosphate buffer was added 40 µl of reaction solution (50 mM sodium phosphate buffer, pH 7.4, 1 mM dithiothreitol) containing a substrate for MTAP, [8-¹⁴C] MTA (final concentration 20 µM) and reacted at room temperature for 60 minutes. After removal of proteins precipitated with 30% (v/v) trichloroacetic acid, 30 µl of the supernatant was applied to thin-layer chromatography eluting with CH₃CN : 50 mM sodium acetate buffer (pH 4.4) = 85:15. FUJIX Bio-Imaging Analyzer BAS 2000 (Fuji Photo Film) was used to determine the intensity of spots corresponding to the produced [¹⁴C] adenine, which was then compared with a known amount of [¹⁴C] adenine used as a standard to calculate enzymatic activity. Enzymatic activity was defined as 1 unit = 1 nmol of product formed/min/mg of protein. As a result, spots of [¹⁴C] adenine were detected from MOLT-4, Raji and U937 but not from CCRF-CEM and K562, as shown in Fig. 1.

### Example 2: Selection procedure of adenylosuccinate synthase inhibitors

As existing adenylosuccinate synthase inhibitors, hydantocidin-5'-monophosphate and hadacidin were chosen and compared for their level of enzyme inhibitory activity. Hydantocidin-5'-monophosphate was synthesized by debenzylating an intermediate synthesized by the method of Mio et al. (Tetrahedron, 47, 2133-2144, 1991), [2R, 3R, 4R, 5S]-6-N-acetyl-2-benzyloxymethyl-3,4-isopropylidene-1-oxa-6,8-diazaspiro[4,4]nonane-7,9-dione (Compound 24) and then converting it into its diphenyl phosphate ester, followed by deacetonidation, deacetylation and dephenylation by catalytic reduction. Hadacidin was synthesized by the method of Jahngen-Rossomand (Synth. Commun. 12, 601-606, 1982). Adenylosuccinate synthase was purified from rabbit muscles by the method of Fischer et al. (Methods in Enzymol. LI, 207-213). Each inhibitor (hydantocidin-5'-monophosphate or hadacidin at a final concentration of 1µM-1mM) (10 µl) and a substrate solution (50 mM HEPES, pH 7.3, 1 mM MgCl₂, 0.2 mM GTP, 0.2 mM IMP, 2.5 mM Asp [each final concentration]) (60 µl) were added to adenylosuccinate synthase (10 µl) added dropwise into a microspectroscopic cell, and mixed together, then the microspectroscopic cell was immediately set in a spectrophotometer BECKMAN DU650 (Beckman) to determine change of absorbance at 285 nm with time for a period of 2 to 5 minutes after the reaction was started. As shown in Fig. 2, hydantocidin-5'-monophosphate and hadacidin inhibited adenylosuccinate synthase activity at IC₅₀ = 3 x 10⁻⁸ M and 1 x 10⁻⁵ M, respectively.

CCRF-CEM cells lacking MTAP activity and Raji cells having MTAP activity suspended in a modified MEM Zn⁺⁺ medium (GIBCO BRL) containing 10% dialyzed horse serum (GIBCO BRL) pretreated at 56°C for 30 minutes were plated on 96-well microplates at concentrations of 1.5 x 10⁵ cells/ml and 5 x 10⁴ cells/ml, respectively, and adenylosuccinate synthase inhibitors (hydantocidin-5'-monophosphate and hadacidin at a final concentration of 1 µM-1mM) were added to 100 µl/well in the presence or absence of a substrate for MTAP, MTA (final concentration 10 µM). After incubation in the presence of 5% CO₂ at 37°C for 2 days, the survival cell number was counted by the MTT method (J. Immunol. Methods, 65, 55-63, 1983) (using Cell Titer 96® Aqueous Non-Radioactive Cell Proliferation Assay [Promega]) to assess effects on cell growth. When MTA was added to promote AMP synthesis in the salvage pathway, hydontocidin-5'-monophosphate did not show cytotoxicity to Raji cells having MTAP activity, but selectively showed cytotoxicity to CCRF-CEM cells lacking MTAP activity (IC₅₀ = 3 x 10⁻⁵ M). The level of this cytotoxicity was comparable to that of each type of cells in the absence of MTA, i.e. when the salvage pathway does not function well (Fig. 3A, C). Hadacidin showed a similar tendency (Fig. 3B, D). Thus selected hydantocidin-5'-monophosphate also showed selective toxicity to a human glioma cell line A-172 lacking MTAP activity (Fig. 4A) and a human glioma cell line T98G having MTAP activity (Fig. 4B). Similarly, hydantocidin-5'-monophosphate also showed growth inhibitory effects on other types of tumor cells known to lack MTAP activity such as a human non-small cell lung cancer cell line A549 (Proc. Natl. Acad. Sci. USA, 93, 6203-6208, 1996) or a human pancreatic cancer cell line BxPC-3 (Cancer Res., 56, 1083-1090, 1996) (Fig. 5A, B).

### INDUSTRIAL APPLICABILITY

Because of their high selectivity for target cells lacking an enzyme essential for the second metabolic pathway of an essential compound, drugs of the present invention have no or little toxicity to normal cells having said enzyme so that they can minimize side effects involving physical and mental pains of patients caused by administration of conventional anticancer drugs or the like.

## Claims

1. A drug containing an enzyme inhibitor as an active ingredient, wherein said enzyme inhibitor satisfies the following requirements:
1) said enzyme inhibitor inhibits at least one enzyme which is essential for one metabolic pathway (first pathway) of a compound essential for cell survival (essential compound);
2) said essential compound is also metabolizable by another pathway (second pathway) which is different from said first pathway and said enzyme inhibitor does not inhibit said second pathway; and
3) at least one enzyme essential for said second pathway is functionally lost in a target cell subjected to growth inhibition by said drug.

2. The drug of Claim 1 which is an anticancer drug.

3. The drug of Claim 1 or 2 wherein the compound essential for cell survival is a nucleotide or a nucleotide metabolite.

4. The drug of any one of Claims 1 to 3 which further comprises a compound serving as a substrate for at least one enzyme essential for the second pathway.

5. The drug of any one of Claims 1 to 4 wherein at least one enzyme essential for the first pathway is adenylosuccinate synthase.

6. The drug of any one of Claims 1 to 5 wherein the enzyme inhibitor is an adenylosuccinate synthase inhibitor.

7. The drug of Claim 6 wherein the adenylosuccinate synthase inhibitor is hydantocidin or a hydantocidin derivative.

8. The drug of Claim 7 wherein the hydantocidin derivative is hydantocidin-5'-monophosphate.

9. The drug of any one of Claims 1 to 8 wherein at least one enzyme essential for the second pathway is 5'-methylthioadenosine phosphorylase.

10. The drug of Claim 4 wherein the compound serving as a substrate for at least one enzyme essential for the second pathway is 5'-methylthioadenosine.

11. The drug of any one of Claims 1 to 10 wherein the functional deficiency in an enzyme is caused by a mutation of the gene for said enzyme.

12. The drug of any one of Claims 1 to 11 wherein the target cell subjected to growth inhibition is a human leukemia cell.

13. The drug of Claim 12 wherein the human leukemia cell is the human leukemia cell line CCRF-CEM.

14. The drug of any one of Claims 1 to 11 wherein the target cell subjected to growth inhibition is a human glioma cell.

15. The drug of Claim 14 wherein the human glioma cell is the human glioma cell line A-172.

16. The drug of any one of Claims 1 to 11 wherein the target cell subjected to growth inhibition is a human non-small cell lung cancer cell.

17. The drug of Claim 16 wherein the human non-small cell lung cancer cell is the human non-small cell lung cancer cell line A549.

18. The drug of any one of Claims 1 to 11 wherein the target cell subjected to growth inhibition is a human pancreatic cancer cell.

19. The drug of Claim 18 wherein the human pancreatic cancer cell is the human pancreatic cancer cell line BxPC-3.

20. A method for testing the potency of a drug containing an enzyme inhibitor as an active ingredient, wherein said enzyme inhibitor inhibits at least one enzyme which is essential for one metabolic pathway (first pathway) of a compound essential for cell survival (essential compound). and said essential compound is also metabolizable by another pathway (second pathway) which is different from said first pathway, and said enzyme inhibitor does not inhibit said second pathway.

21. The method of Claim 20 comprising determining that at least one enzyme essential for the second pathway of a compound essential for cell survival (essential compound) is functionally lost in a target cell subjected to growth inhibition by said drug.

22. The method of Claim 20 or 21 wherein the drug is an anticancer drug.

23. The method of any one of Claims 20 to 22 wherein the compound essential for cell survival is a nucleotide or a nucleotide metabolite.

24. The method of any one of Claims 20 to 23 wherein the enzyme inhibitor is an adenylosuccinate synthase inhibitor.

25. The method of Claim 24 wherein the adenylosuccinate synthase inhibitor is hydantocidin or a hydantocidin derivative.

26. The method of any one of Claims 20 to 25 wherein at least one enzyme essential for the second pathway is 5'-methylthioadenosine phosphorylase.

27. The method of any one of Claims 20 to 26 wherein the functional deficiency in an enzyme is caused by a mutation of the gene for said enzyme.

28. The method of any one of Claims 20 to 27 wherein the target cell subjected to growth inhibition is a human leukemia cell.

29. The method of Claim 28 wherein the human leukemia cell is the human leukemia cell line CCRF-CEM.

30. The method of any one of Claims 20 to 27 wherein the target cell subjected to growth inhibition is a human glioma cell.

31. The method of Claim 30 wherein the human glioma cell is the human glioma cell line A-172.

32. The method of any one of Claims 20 to 27 wherein the target cell subjected to growth inhibition is a human non-small cell lung cancer cell.

33. The method of Claim 32 wherein the human non-small cell lung cancer cell is the human non-small cell lung cancer cell line A549.

34. The method of any one of Claims 20 to 27 wherein the target cell subjected to growth inhibition is a human pancreatic cancer cell.

35. The method of Claim 34 wherein the human pancreatic cancer cell is the human pancreatic cancer cell line BxPC-3.

36. A kit for testing the potency of a drug containing an enzyme inhibitor as an active ingredient, wherein said enzyme inhibitor inhibits at least one enzyme which is essential for one metabolic pathway (first pathway) of a compound essential for cell survival (essential compound), and said essential compound is also metabolizable by another pathway (second pathway) which is different from said first pathway, and said enzyme inhibitor does not inhibit said second pathway.

37. The kit of Claim 36 for determining that at least one enzyme essential for the second pathway of a compound essential for cell survival (essential compound) is functionally lost in a target cell subjected to growth inhibition by said drug.

38. The kit of Claim 36 or 37 comprising a primer consisting of an oligo DNA fragment corresponding to the DNA sequence of a gene encoding at least one enzyme essential for the second pathway of a compound essential for cell survival (essential compound).
